Europäisches Patentamt

**European Patent Office**

**Office européen des brevets**

(19)

(11) Publication number: **0 232 233**
A1

(12) # EUROPEAN PATENT APPLICATION

(21) Application number: **87850002.4**

(22) Date of filing: **07.01.87**

(51) Int. Cl.³: **A 61 M 5/14**
**F 16 K 7/06**

(30) Priority: **07.01.86 SE 8600042**

(43) Date of publication of application:
**12.08.87 Bulletin 87/33**

(84) Designated Contracting States:
**DE FR GB IT SE**

(71) Applicant: **Mediplast AB**
**Box 1326**
**S-171 26 Solna(SE)**

(72) Inventor: **Ekholmer, Erik**
**Hammarbacken 1**
**S-182 35 Danderyd(SE)**

(74) Representative: **Roth, Ernst Adolf Michael et al,**
**GÖTEBORGS PATENTBYRA AB Box 5005**
**S-402 21 Göteborg(SE)**

(54) **A tube clamp for regulating the flow area of a flexible tube.**

(57) A tube clamp comprising a clamp housing (1) through which the tube (11) extends, a clamping member (9) and a press plate (5) arranged between the tube and the clamping member. The press plate (5) is connected to the clamp housing (1) in such a way that it will be displaced towards and away from the tube when actuated by the clamping member. The clamping member may consist of a roller (9) with axle necks (8a,8b) which are guided in inclined grooves (4) in the clamp housing. The press plate (5) has a substantially U-shaped cross-section with side gables (7a,7b), on the upper edges of which the clamping member (9) is arranged to act and which side gables are guided against the inside of the clamp housing so that the press plate (5) is displaced towards and away from the clamp surface of the clamp housing when actuated by the clamping member (9).

FIG 2a

FIG 2b

FIG 3

EP 0 232 233 A1

A TUBE CLAMP FOR REGULATING THE FLOW AREA OF A FLEXIBLE TUBE

Technical field

The present invention refers to a tube clamp for regulating the flow area of a flexible tube and of the kind comprising a clamp housing through which the tube is intended to extand and which has a clamp surface against which the tube is supported, a clamping member arranged to press the tube against said surface and a press plate which is substantially fixed against displacement in the axial direction of the tube but movable towards and away from the tube. At first hand but not exclusively the tube clamp is intended for medical applications as for regulating the flow of infusion solutions or the like through a tube.

Background of the invention

So called drop regulators are previously known through for example the Swedish patent application No. 8306127-5 which discloses a device where a clamp housing, through which the tube is passed, is provided with a longitudinal groove with a gradually increasing width and a gradually decreasing depth towards the outlet end of the tube. Through such an arrangement the tube is given a correct guidance and is permitted to be deformed in a correct way since the width and the depth at each section are adapted after the shape of the tube at the section in question. It can however not be avoided that the loads on the material becomes very irregular and large especially in the outer portions of the tube, especially since the point or at best the line contact which occurs between the roller and the tube is limited to a very small zone.

A further drawback is the cold flow which occurs in the plastic material of the tube at the displacement of the roller and which makes an exact regulation of the flow area of the tube difficult and decreases the ability of the tube material to revert to its original shape and dimension after the compression.

Through for example GB-A-1.506.895, US-A-4.285.492 and GB-A-2.042.131 it is previously known to arrange a press plate between the clamping member and the tube in order to provide a more even distribution of the compression pressure. The clamping member acts directly against the press plate.

## The purpose and most important features of the invention

The object of the present invention is to provide a device of the kind mentioned above, which minimizes the cold flow tendency and provides a more even distribution of the compression pressure and besides a gear effect between the clamping member and the press plate. By that a more exact and more reliable adjustment of the drop rate, especially at high compression degrees, i.e. small drop rates, is possible.

A further object is to provide a device which on one hand is durable and reliable and on the other hand can be manufactured to such a low price that it can be disposed after use.

These objects have according to the invention been achieved by the fact that the press plate has a substantially U-shaped cross section with upstanding side gables, a clamping member being arranged to act upon the upper edges of said side gables, which are guided against the inside of the clamp housing so that the press plate when actuated by the clamping member is displaced towards or away from the clamp surface of the clamp housing.

## Description of the drawings

The invention will now be closer described with reference to the accompanying drawings showing a pair of embodiments of the invention.

Figure 1 is a central longitudinal section through a first embodiment of the invention,

Figures 2a and 2b are cross sections according to the line IIa-IIa and IIb-IIb resp. in figure 1,

Figure 3 is a perspective view of the injection moulded article of which the clamp housing according to figure 1 and 2 consists,

Figure 4 is a longitudinal section through a second embodiment of the invention,

Figure 5 is a perspective view of the press plate according to figure 1, and

Figure 6 is a perspective view of the clamp housing according to figure 4 and 5.

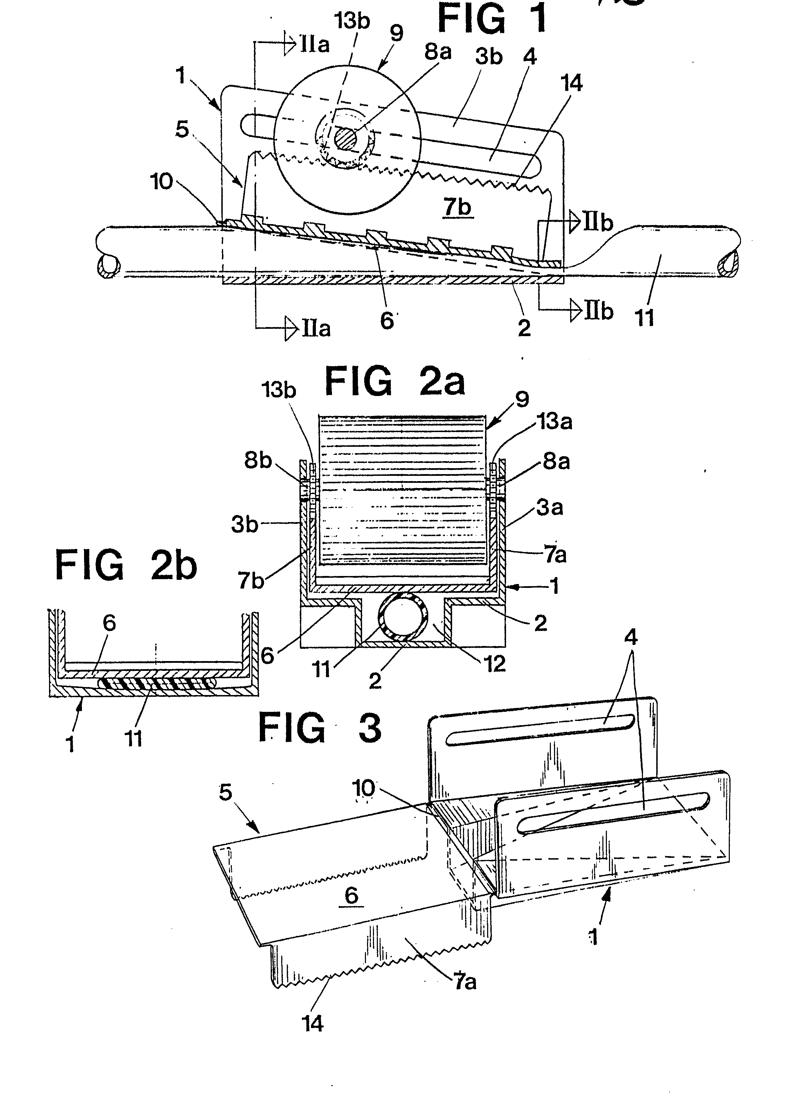

The embodiment shown in figures 1-3 comprises a clamp housing 1, which has a substantially U-shaped cross section. The clamp housing has a rib portion acting as a clamp surface 2 and two spaced upstanding shanks 3a, 3b which are parallel and which preferably are relatively thin-walled and resilient. Each shank is provided with an elongated opening 4 which is inclined with respect to the clamp surface.

The device further comprises a press plate 5, which like the clamp housing, has a substantially U-shaped cross section comprising a rib portion acting as a clamp surface 6 and upstanding side gables 7a, 7b. The device also comprises a press roller 9 provided with short axle necks 8a, 8b. The axle necks 8a, 8b are provided with cog wheels 13a, 13b cooperating with cogs 14 on the side gables 7a, 7b. The cog action between the roller 9 and the side gables 7a, 7b of the press plate prevents slipping of the roll 9 and assures guidance of the roller 9 with respect to the press plate 5.

The clamp housing as well as the press plate and the press roller are preferably manufactured of plastic material and as can be seen from figure 3 the clamp housing and the press plate can be made as one integral piece by injection moulding, at which figure 3 shows the article provided through the injection moulding. This is provided with a thin connection portion 10 and by an appropriate choice of plastic material, for example polypropylene, it can be obtained that the press plate 5 can be folded or bent in the thin connection portion 10 so that the press plate can take the position shown in figure 1. Polypropylene has an inherent hinge effect, at which the press plate in this case will be hingedly connected to the clamp housing. It is however possible to use materials which only permits folding, materials which provides a resilient effect etc. As can be seen from Fig. 2 the clamp surface 2 in the shown embodiment consists of the bottom of a groove, which has a decreasing depth from the inlet side of the tube, i.e. to the left of figure 1, and towards the outlet side of the tube. At the same time the width of the groove increases gradually in the same direction and the relation width/depth corresponds to the dimensions of the tube at the compression thereof in different positions of the press roller.

In the embodiment shown the shanks 3a, 3b of the clamp housing 1 are relatively thin-walled and resilient so that they can be brought apart to some degree. Due to the fact that the axle necks of the press roller are short and rounded or chamferred at the ends they can be snapped into the elongated openings 4.

In order to put together the device the press plate 5 is firstly folded substantially $180^0$ from the position shown in figure 3, after which the press roller is applied by snapping its axle necks into the openings 4.

When the tube 11 has been passed through the clamp housing the press roller is brought from its left, inoperative position, to the right. The inclined openings will then gradually approach the clamp surface 2 and by that the press plate is pivoted towards the clamp surface 2 with the connection portion 10 as pivot center. By that the tube 11 is compressed and its flow area is reduced correspondingly. Due to the fact that the press roller 9 acts upon the upper edge of the side gables 7a, 7b of the press plate 6 a certain gear effect is provided between the roller 9 and the press plate 6, at which the compression of the tube 2 at high degrees of compressions is facilitated.

The drawbacks of cold flow mentioned above are most common when the flow area has been strongly reduced. In order to provide a more even distribution of pressure in such cases the press plate 5 can be so designed that it is given a certain flexibility close to its free end. When tightened the clamp surface 6 can therefore according to Fig. 1 be somewhat bent at which the compression is distributed instead of being concentrated to one single point.

For this reason the groove 12 in the clamp housing should at least in its shallow portion be somewhat shallower than the theoretically correct. Due to the fact that the compressed portion of the tube obtains a relatively great length it will also be possible to improve the sealing by arranging small ridges or the like on the surfaces 2 and 6 which act on the tube. These ridges can provide a labyrinth-like compressions of the tube.

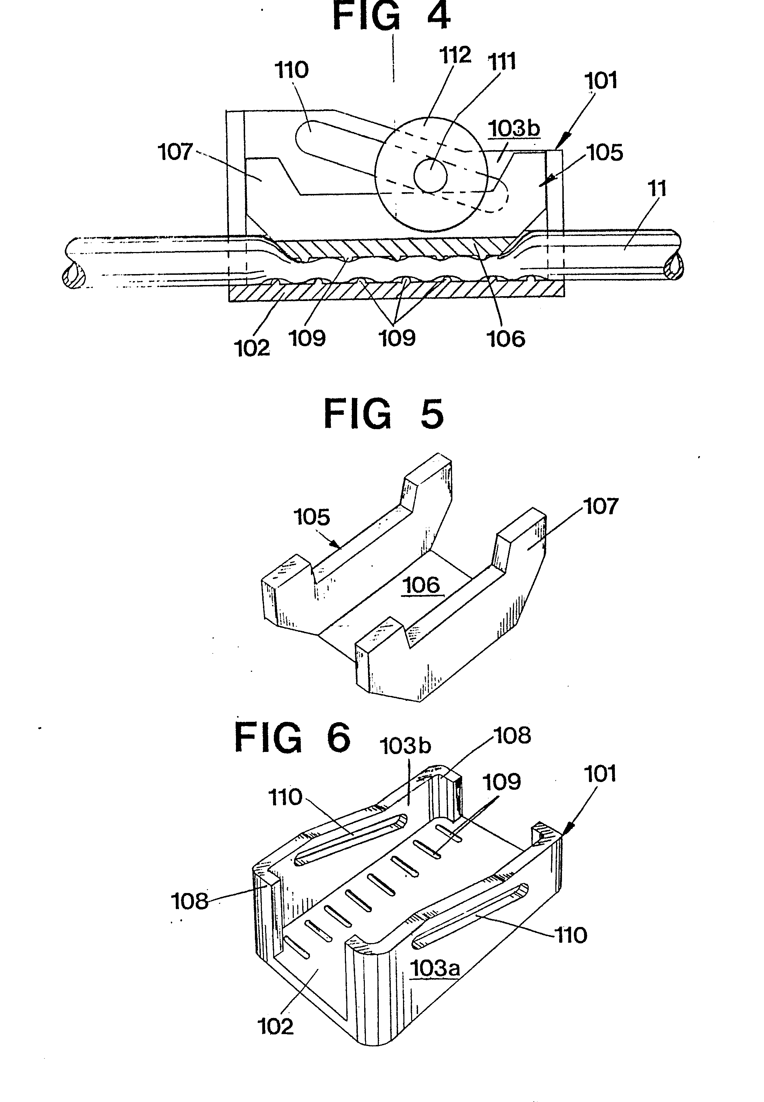

The embodiment disclosed in figure 4-6 differs from the one described above substantially by the fact that the press plate is displaceably arranged with respect to the clamp housing in a direction perpendicular to the longitudinal direction of the tube instead of being pivotally connected to the clamp housing.

The clamp housing 101 has a plane contact surface 102 for the tube 11 and upstanding side portions 103a, 103b, which like the shanks 3a, 3b has inclined openings 110 with respect to the contact surface 102 for guiding axle necks 111 to a press roller 112. The press plate 106 has a clamp surface 106 and upstanding side gables 107, the upper edges of which cooperate with the axle necks 111 of the press roller 112. The side gables 107 also improves the rigidity of the press plate and provides a guidance against the clamp housing, so that the press plate can be displaced in a direction towards or away from the tube but not in the longitudinal direction thereof. For this purpose the clamp housing has gable portions 108 cooperating with the side gables 107.

Also in this case it is obtained that the compression takes place along a relatively great length and it can therefore also here be possible to arrange ridges 109 or the like for improving the sealing, especially at the outer portions of the tube. Through such sealings and the longer compression length the stresses of the tube material is minimized.

The invention is of course not limited to the embodiments described above and shown in the drawings but a plurality of modifications are possible within the scope of the claims.

CLAIMS

1. A tube clamp for regulating the flow area of a flexible tube and of the kind comprising a clamp housing (1;101) through which the tube (11) is intended to extend and which has a clamp surface (2;102) against which the tube (11) is supported, a clamping member (9;112) arranged to press the tube against said surface and a press plate (5;105) which is substantially fixed against displacement in the axial direction of the tube but movable towards and away from the tube,

c h a r a c t e r i z e d   i n,

that the press plate (5;105) has a substantially U-shaped cross-section with upstanding side gables (7a,7b;107), the clamping member (9) being arranged to act upon the upper edges of said side gables, which are guided against the inside of the clamp housing (1;101) so that the press plate (5;105) when actuated by the clamping member (9) is displaced towards or away from the clamp surface (2;102) of the clamp housing (1;101).

2. A tube clamp according to claim 1,

c h a r a c t e r i z e d   i n,

that the clamp housing (1) and the press plate (5) are manufactured of plastic material preferably through injection moulding as an integral unit interconnected by a bendable or foldable connection portion (10).

3. A tube clamp according to claims 1 or 2,

c h a r a c t e r i z e d   i n,

that the clamping member (9) consists of a roller the axle necks of which is provided with cog wheels (13a, 13b) cooperating with cogs (14) on the side gables (7a,7b) of the press plate (5).

4. A tube clamp according to one or more of the preceding claims, in which the clamp surface (2) consists of the bottom of a longitudinal groove (12) arranged in the bottom of the lamp housing (1), in which the depth of said groove gradually decreases and the width gradually increases towards the outlet end of the tube for adaption to the cross section of the gradually compressed tube,

characterized in,

that said groove (12) at least in its shallow portion is somewhat shallower than theoretically correct, at which the press plate (5) at least in the portion where the compression reaches a maximum is flexible in order to provide a distribution of the pressing pressure along the longitudinal direction of the tube.

5. A tube clamp according to any of the preceding claims, characterized in,

that the clamp surface (102) of the clamp housing (101) and/or the press plate (105) is provided with ridges (109) or the like arranged in connection to the outer portions of the tube and intended to provide a preferably labyrinth-like sealing.

6. A tube clamp according to any of the preceding claims, in which the clamp housing (1;101) has a substantially U-shaped cross-section,

characterized in,

that the shanks (3a,3b;103a,103b) of the clamp housing are somewhat resilient, that the clamping member consists of a roller (9;112) provided with axle necks (8;111), which are short and preferably provided with chamferred or rounded end portions in order to make it possible to insert said axle necks in elongated inclined grooves (4;110) in said shanks through snap action.

# FIG 1

# FIG 2a

# FIG 2b

# FIG 3

# FIG 4

# FIG 5

# FIG 6

European Patent Office

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | US-A-3 915 167 (WATERMAN) --- | | A 61 M 5/14<br>F 16 K 7/06 |
| A | FR-A- 682 386 (CHRISTIANSEN) --- | | |
| A | US-A-2 112 625 (JACKSON) ----- | | |

**TECHNICAL FIELDS SEARCHED (Int. Cl 4)**

F 16 K
A 61 M

The present search report has been drawn up for all claims

| Place of search | Date of completion of the search | Examiner |
|---|---|---|
| THE HAGUE | 03-04-1987 | VERELST P.E.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82